(19) Europäisches Patentamt
European Patent Office
Office européen des brevets

(11) Veröffentlichungsnummer: **0 230 056**
**B1**

(12) # EUROPÄISCHE PATENTSCHRIFT

(45) Veröffentlichungstag der Patentschrift: 08.02.89

(51) Int. Cl.⁴: C 07 F 9/38, C 07 F 9/30, G 01 N 33/78

(21) Anmeldenummer: 86118188.1

(22) Anmeldetag: 31.12.86

ERRATUM

| DIE TEXTSTELLE : TEXT PUBLISHED : LE PASSAGE SUIVANT : | (SEITE, SPALTE, ZEILE) (PAGE, COLUMN, LINE) (PAGE, COLONNE, LIGNE) | | | LAUTET BERICHTIGT: SHOULD READ : DEVRAIT ETRE LU : |
|---|---|---|---|---|
| der Formel III ($R^3$=H) | 4 | 5 | 26 | der Formel III ($R^3 \neq$ H) |
| der Formel III ($R^3$=H) | 4 | 5 | 27 | der Formel III ($R^3 \neq$ H) |

Tag der Entscheidung über die Berichtigung / Date of decision on rectification: / Date de décision portant ...: 03.04.89

Ausgabe- und Veröffentlichungstag: / Issue and publication date: / Date d'edition et de publication: 31.05.89

Patbl.Nr / EPB no: / Bull. no: 89/2

(19) Europäisches Patentamt
European Patent Office
Office européen des brevets

(11) Veröffentlichungsnummer: **0 230 056**
**B1**

(12) # EUROPÄISCHE PATENTSCHRIFT

(45) Veröffentlichungstag der Patentschrift: **08.02.89**

(51) Int. Cl.4: **C 07 F 9/38**, C 07 F 9/30, G 01 N 33/78

(21) Anmeldenummer: **86118188.1**

(22) Anmeldetag: **31.12.86**

(54) **Neue Thyroninderivate.**

(30) Priorität: **09.01.86 DE 3600365**

(43) Veröffentlichungstag der Anmeldung:
**29.07.87 Patentblatt 87/31**

(45) Bekanntmachung des Hinweises auf die Patenterteilung:
**08.02.89 Patentblatt 89/6**

(84) Benannte Vertragsstaaten:
**AT BE CH DE ES FR GB GR IT LI LU NL SE**

(56) Entgegenhaltungen:
**FR-A- 2 317 655**
**US-A- 4 480 041**

(73) Patentinhaber: **HOECHST AKTIENGESELLSCHAFT, Postfach 80 03 20, D-6230 Frankfurt am Main 80 (DE)**

(72) Erfinder: **Wissmann, Hans, Dr., Falkenstrasse 12, D-6232 Bad Soden am Taunus (DE)**
Erfinder: **Hachmann, Henning, Dr., Village Circle 1, Lexington MA. 02173 (US)**
Erfinder: **Simons, Guido, Dr., Talstrasse 24, D-6507 Ingelheim am Rhein (DE)**
Erfinder: **Strecker, Helmut, Dr., Odenwaldstrasse 56, D-6102 Pfungstadt (DE)**

**EP 0 230 056 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Thyroninderivate der allgemeinen Formel I,

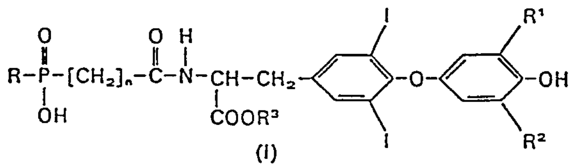

(I)

in welcher
n für eine ganze Zahl zwischen 1 und 6 steht,
R Hydroxy, $(C_1\text{-}C_6)$-Alkyl oder $(C_6\text{-}C_{10})$-Aryl bedeutet,
$R^1$ und $R^2$ gleich oder verschieden sind und Iod oder Wasserstoff bedeuten und
$R^3$ Wasserstoff, $(C_1\text{-}C_6)$-Alkyl oder $(C_7\text{-}C_{10})$-Aralkyl bedeutet,
sowie deren Salze.

Bevorzugt sind Verbindungen der Formel I, in welcher $R^1$ und $R^2$ wie oben definiert sind und n = 1 bis 4 ist, R Hydroxy oder $(C_1\text{-}C_4)$-Alkyl und $R^3$ Wasserstoff oder $(C_1\text{-}C_4)$-Alkyl, insbesondere Wasserstoff, Methyl oder Ethyl bedeuten.

Alkyl kann geradkettig oder verzweigt sein. Unter $(C_7\text{-}C_{10})$-Aralkyl wird z.B. Benzyl oder Phenethyl, vorzugsweise Benzyl verstanden. $(C_6\text{-}C_{10})$-Aryl bedeutet vorzugsweise Phenyl.

Unter Salzen der Verbindungen der Formel I werden insbesondere Alkalimetall-, Erdalkalimetall- und Ammoniumsalze verstanden.

Die Erfindung betrifft weiterhin ein Verfahren zur Herstellung von Verbindungen der Formel I, das dadurch gekennzeichnet ist, dass man Verbindungen der Formel II,

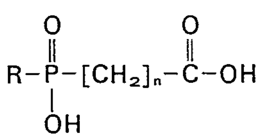

(II)

in welcher R und n wie oben definiert sind, oder deren aktiviertes Derivat mit Verbindungen der Formel III,

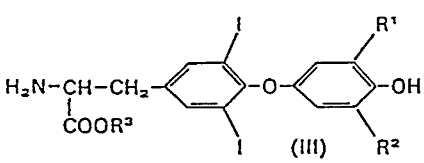

(III)

in welcher $R^1$, $R^2$ und $R^3$ wie oben definiert sind und $R^3$ vorzugsweise = H ist, umsetzt oder gegebenenfalls Ester der Formel III ($R^3 \neq H$) einsetzt, erhaltene Ester der Formel I ($R^3 \neq H$) gegebenenfalls in die freie Säure der Formel I ($R^3$ = H) überführt und die so erhaltenen Verbindungen der Formel I gegebenenfalls in ihre Salze überführt.

Als Methoden zur Herstellung der Amidbindung zwischen den Tyroninestern der Formel III und den Phosphino- bzw. Phosphonoalkancarbonsäuren der Formel II können alle gängigen Peptidsynthesemethoden zur Anwendung kommen, wie beispielsweise die Carbodiimidmethode (siehe z.B. Schröder, Lübke, The Peptides, Volume I, Academic Press New York, London 1963, Seiten 108-111), die Methode der gemischten Anhydride (siehe z.B. ebenda, Seiten 77-97) sowie die Methoden unter Verwendung von Alkylphosphonsäureanhydriden bzw. Dialkylphosphinsäureanhydriden (Kleiner, Wissmann, Angew. Chem. *92* [1980] 129 bzw. EP-A-56 618).

Aus den Estern der Formel I kann die Verbindung mit freier Carboxylgruppe in an sich bekannter Weise durch Hydrolyse oder Hydrogenolyse freigesetzt werden. Bevorzugt ist die Verseifung der Niederalkylester mit gemischt-wässerigen Alkalien.

Die erfindungsgemässen Verbindungen zeichnen sich durch spezifische, für den Radioimmunoassay von Thyroninderivaten günstige Löslichkeits-, Adsorptions- und Bindungseigenschaften, aus.

Verbindungen der Formel I können in üblicher Weise (z.B. durch Iodaustausch oder Iodierung) radioaktiv markiert werden. Bevorzugt ist die Markierung mit dem Isotop $^{125}I$.

Nachstehende Beispiele dienen zur Erläuterung der vorliegenden Erfindung, ohne dass diese darauf beschränkt wäre.

1) *Methylphosphinoacetyl-tetraiodthyronin*

In einer Mischung aus 2,5 ml Dimethylformamid und 2,5 ml Methanphosphonsäure-bis-dimethylamid löst man 160 mg (1,16 Millimol) Methylphosphinoessigsäure und 0,4 ml (3,16 Millimol) N-Ethylmorpholin. Dann tropft man unter Eiskühlung 0,4 ml (2,32 Millimol) Methylethylphosphinsäureanhydrid zu. Man rührt 10 min bei Raumtemperatur nach und gibt anschliessend 800 mg (1,03 Millimol) Thyroxin (Tetraiodthyronin) zu. Die Reaktionslösung lässt man über Nacht bei Raumtemperatur stehen, dann fällt man das Reaktionsprodukt durch Zugabe von Wasser und ansäuern mit verdünnter wässeriger HCl auf pH = 3.

Ausbeute an Rohprodukt: 920 mg.

Die Dünnschichtchromatographie (DC) des Produktes (Kieselgel 60, (Merck) Lösungsmittel $CHCl_3/CH_3OH$/Eisessig, 100:20:2) zeigt, dass das Ausgangsmaterial Tetraiodthyronin vollständig umgesetzt ist. Die Reinigung des Rohproduktes erfolgt über eine Kieselgel 60-Säule (4,5 × 35 cm) mit dem Lösungsmittelsystem n-Butanol/3,3% $NH_4OH/CH_3OH$ (100:20:2). Das als schwachgefärbtes amorphes Pulver anfallende gereinigte Produkt zeigt einen $R_f$-Wert von 0,75 in dem obengenannten Chloroformsystem. Das $^1H$-NMR-Spektrum zeigt die erwarteten Charakteristika.

2) *Methylphosphinopropionyl-triiodthyroninmethylester*

Zu der Lösung von 110 mg Methylphosphinopropionsäure (0,72 Millimol) und 0,3 ml (2,35 Millimol) N-Ethylmorpholin in 2 ml Dimethylformamid gibt man unter Rühren, Eiskühlung und Feuchtigkeitsausschuss nacheinander 360 mg (0,52 Millimol) Triiodthyroninmethylesterhydrochlorid und 220 mg (1,07 Millimol) Dicyclohexylcarbodiimid, gelöst in 0,5 ml Dimethylformamid, zu. Man lässt unter Rühren auf Raumtemperatur erwärmen, und saugt dann nach 28-stündigem Stehen unter Lichtausschluss bei Raumtemperatur vom ausgefallenen Dicyclohexylharnstoff ab. Den nach Abdestillieren des Lösungsmittels verbleibenden Rückstand fällt man mehrfach aus Ethanol/Äther um.

Ausbeute an Rohprodukt: 365 mg.

3) *Methylphosphinopropionyl-triiodthyronin*

348 mg des Rohproduktes aus Beispiel (2) löst man in 3 ml Methanol und rührt die Lösung nach Zugabe von 2n wässeriger Natronlauge 4 Stunden lang bei pH 12,5 unter Konstanthalten des pH-Wertes mit der Lauge. Anschliessend dampft man aus der mit verdünnter wässeriger Salzsäure neutralisierten Reaktionsmischung das Methanol im Vakuum ab, nimmt in Wasser auf und säuert die Suspension unter Rühren mit 2n wässerigem HCl auf pH 2,5 an. Der Niederschlag wird abgesaugt, mit Wasser gewaschen und im Vakuum über Phosphorpentoxyd getrocknet.

Ausbeute: 205 mg.

Das Produkt wurde durch Elementaranalyse und $^1H$-NMR als solches charakterisiert, die Kieselgel-Dünnschichtchromatographie in mehreren Systemen zeigte die Abwesenheit der Ausgangsmaterialien.

4) *Phosphonoacetyl-diiodthyronin*

Die Herstellung erfolgt wie im Beispiel (2) beschrieben. Das Produkt wurde nach Verseifung durch Säulenchromatographie auf Kieselgel im System $CHCl_3/CH_3OH/H_2O$/Eisessig 100:45:6:1,5 gereinigt, und $^1H$-NMR-spektroskopisch identifiziert.

5) *Methylphosphinoacetyl-diiodthyronin*

Die Verbindung wurde analog der in den Beispielen (2) und (3) beschriebenen Verfahrensweise hergestellt. Anstelle der Methylphosphinopropionsäure wurde die Methylphosphinoessigsäure, und anstelle des Triiodthyroninmethylester-hydrochlorids das Diiodthyroninmethylester-hydrochlorid verwendet. Das Endprodukt wurde durch Inversed Phase-Säulenchromatographie an Kieselgel RP 18 mit 75%igem Methanol als Elutionsmittel gereignigt. Die einheitliche und im DC sich von den Ausgangsmaterialien unterscheidende Substanz der Hauptfraktion wurde durch Massenspektrometrie als die Titelverbindung identifiziert.

**Patentansprüche** für die Vertragsstaaten: BE, CH, DE, FR, GB, IT, LI, LU, NL, SE

1. Verbindung der Formel I,

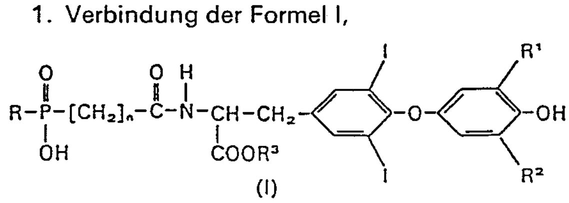

(I)

in welcher

n für eine ganze Zahl zwischen 1 und 6 steht,

R Hydroxy, $(C_1-C_6)$-Alkyl oder $(C_6-C_{10})$-Aryl bedeutet,

$R^1$ und $R^2$ gleich oder verschieden sind und Iod oder Wasserstoff bedeuten und

$R^3$ Wasserstoff, $(C_1-C_6)$-Alkyl oder $(C_7-C_{10})$-Aralkyl bedeutet,

sowie deren Salze.

2. Verbindung der Formel I gemäss Anspruch 1, in welcher

n = 1 bis 4 ist,

R Hydroxy oder $(C_1-C_4)$-Alkyl und

$R^3$ Wasserstoff oder $(C_1-C_4)$-Alkyl bedeuten.

3. Verbindung der Formel I gemäss Anspruch 1 oder 2, in welcher $R^3$ Wasserstoff, Methyl oder Ethyl bedeutet.

4. Verbindung der Formel I gemäss einem der Ansprüche 1-3, in welcher mindestens eines der Iod-Atome radioaktiv als $^{125}I$ markiert ist.

5. Verfahren zur Herstellung einer Verbindung der Formel I gemäss Anspruch 1, dadurch gekennzeichnet, dass man eine Verbindung der Formel II,

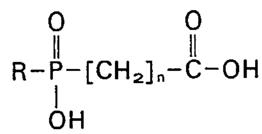

(II)

in welcher R und n wie im Anspruch 1 definiert sind, oder deren aktiviertes Derivat mit einer Verbindung der Formel III,

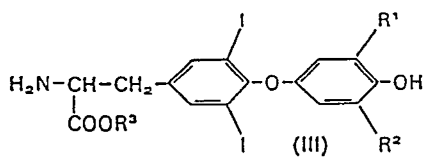

(III)

in welcher $R^1$, $R^2$ und $R^3$ wie im Anspruch 1 definiert sind, umsetzt oder gegebenenfalls einen Ester der Formel III ($R^3 \neq H$) einsetzt, erhaltene Ester der Formel I ($R^3 \neq H$) gegebenenfalls in die freie Säure der Formel I ($R^3$ = H) überführt

und gegebenenfalls die so erhaltene Verbindung der Formel I in ihre Salze überführt.

6. Verwendung einer Verbindung gemäss einem der Ansprüche 1-4 bei der Durchführung eines Radioimmunoassays.

**Patentansprüche** für die Vertragsstaaten: AT, ES, GR

1. Verfahren zur Herstellung einer Verbindung der Formel I,

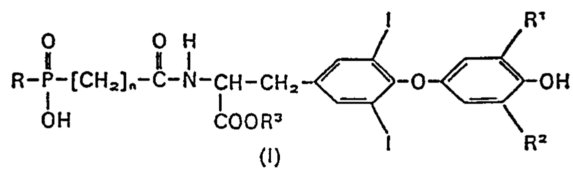

(I)

in welcher

n für eine ganze Zahl zwischen 1 und 6 steht,

R Hydroxy, $(C_1\text{-}C_6)$-Alkyl oder $(C_6\text{-}C_{10})$-Aryl bedeutet,

$R^1$ und $R^2$ gleich oder verschieden sind und Iod oder Wasserstoff bedeuten und

$R^3$ Wasserstoff, $(C_1\text{-}C_6)$-Alkyl oder $(C_7\text{-}C_{10})$-Aralkyl bedeutet,

sowie deren Salze, dadurch gekennzeichnet, dass man eine Verbindung der Formel II,

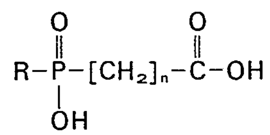
(II)

in welcher R und n wie im Anspruch 1 definiert sind, oder deren aktiviertes Derivat mit einer Verbindung der Formel III,

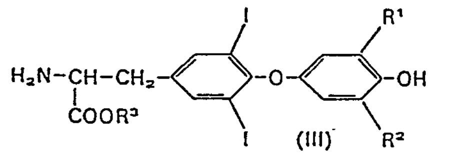
(III)

in welcher $R^1$, $R^2$ und $R^3$ wie im Anspruch 1 definiert sind, umsetzt oder gegebenenfalls einen Ester der Formel III ($R^3$ = H) einsetzt, erhaltene Ester der Formel I ($R^3$ = H) gegebenenfalls in die freie Säure der Formel I ($R^3$ = H) überführt

und gegebenenfalls die so erhaltene Verbindung der Formel I in ihre Salze überführt.

2. Verfahren gemäss Anspruch 1, dadurch gekennzeichnet, dass eine Verbindung der Formel I hergestellt wird, in welcher

n = 1 bis 4 ist,

R Hydroxy oder $(C_1\text{-}C_4)$-Alkyl und

$R^3$ Wasserstoff oder $(C_1\text{-}C_4)$-Alkyl bedeuten.

3. Verfahren gemäss Anspruch 1 oder 2, dadurch gekennzeichnet, dass eine Verbindung der Formel I hergestellt wird, in welcher $R^3$ Wasserstoff, Methyl oder Ethyl bedeutet.

4. Verfahren gemäss einem der Ansprüche 1-3, dadurch gekennzeichnet, dass eine Verbindung der Formel I hergestellt wird, in welcher mindestens eines der Iod-Atome radioaktiv als $^{125}I$ markiert ist.

5. Verwendung einer Verbindung, hergestellt gemäss einem der Ansprüche 1-4, bei der Durchführung eines Radioimmunoassays.

**Claims** for the contracting states: BE, CH, DE, FR, GB, IT, LI, LU, NL, SE

1. A compound of the formula I,

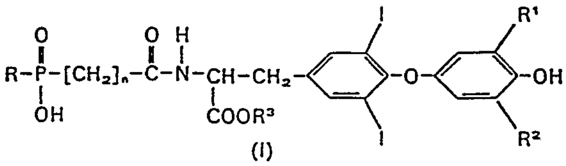
(I)

in which

n represents an integer between 1 and 6,

R denotes hydroxyl, $(C_1\text{-}C_6)$-alkyl or $(C_6\text{-}C_{10})$-aryl,

$R^1$ and $R^2$ are identical or different and denote iodine or hydrogen, and

$R^3$ denotes hydrogen, $(C_1\text{-}C_6)$-alkyl or $(C_7\text{-}C_{10})$-aralkyl,

and the salts thereof.

2. A compound of the formula I as claimed in claim 1, which

R denotes hydroxyl or $(C_1\text{-}C_4)$-alkyl, and

$R^3$ denotes hydrogen or $(C_1\text{-}C_4)$-alkyl.

3. A compound of the formula I as claimed in claim 1 or 2, in which $R^3$ denotes hydrogen, methyl or ethyl.

4. A compound of the formule I as claimed in one of claims 1-3, in which at least one of the iodine atoms is radioactively labeled as $^{125}I$.

5. A process for the preparation of a compound of the formula I as claimed in claim 1, which comprises reacting a compound of the formula II,

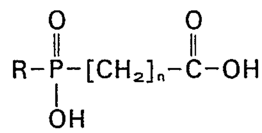
(I)

in which R and n are as defined in claim 1, or an activated derivative thereof, with a compound of the formula III,

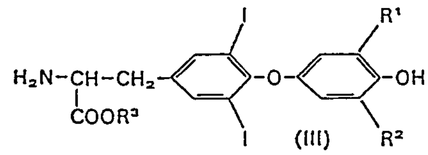
(III)

in which $R^1$, $R^2$ and $R^3$ are as defined in claim 1, or, if appropriate, employing an ester of the formula III ($R^3 \neq$ H), converting the resultant esters of the formula I ($R^3 \neq$ H), if appropriate, into the free acid of the formula I ($R^3$ = H), and, if appropriate, converting the compound of the formula I thus obtained into the salts thereof.

6. The use of a compound as claimed in one of claims 1-4 for carrying out a radioimmunoassay.

**Claims** for the contracting states: AT, ES, GR

1. A process for the preparation of a compound of the formula 1,

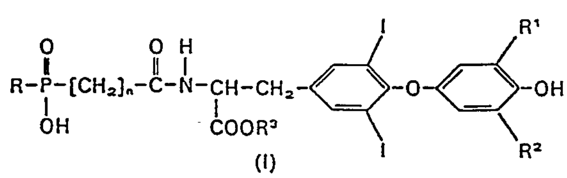
(I)

in which

n represents an integer between 1 and 6,

R denotes hydroxyl, $(C_1\text{-}C_6)$-alkyl or $(C_6\text{-}C_{10})$-aryl,

$R^1$ and $R^2$ are identical or different and denote iodine or hydrogen, and

$R^3$ denotes hydrogen, $(C_1\text{-}C_6)$-alkyl or $(C_7\text{-}C_{10})$-aralkyl,

and the salts thereof, which comprises reacting a compound of the formula II,

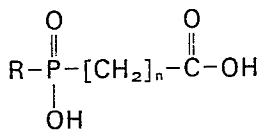

(II)

in which R and n are as defined in claim 1, or an activated derivative thereof, with a compound of the formula III,

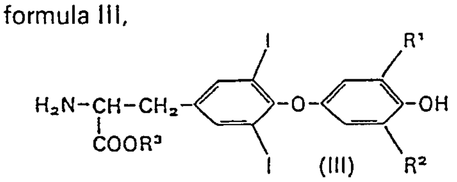

(III)

in which $R^1$, $R^2$ and $R^3$ are as defined in claim 1, or, if appropriate, employing an ester of the formula III ($R^3 \neq H$), converting the resultant esters of the formula I ($R^3 \neq H$), if appropriate, into the free acid of the formula I ($R^3 = H$), and, if appropriate, converting the compound of the formula I thus obtained into the salts thereof.

2. The process as claimed in claim 1, wherein a compound of the formula I is prepared, in which
n is 1 to 4,
R denotes hydroxyl or $(C_1-C_4)$-alkyl, and
$R^3$ denotes hydrogen or $(C_1-C_4)$-alkyl.

3. The process as claimed in claim 1 or 2, wherein a compound of the formula I is prepared, in which $R^3$ denotes hydrogen, methyl or ethyl.

4. The process as claimed in one of claims 1-3, wherein a compound of the formula I is prepared, in which at least one of the iodine atoms is radioactively labeled as $^{125}I$.

5. The use of a compound prepared as claimed in one of claims 1-4 for carrying out a radioimmunoassay.

**Revendications** pour les Etats contractants: BE, CH, DE, FR, GB, IT, LI, LU, NL, SE

1. Composé de formule I

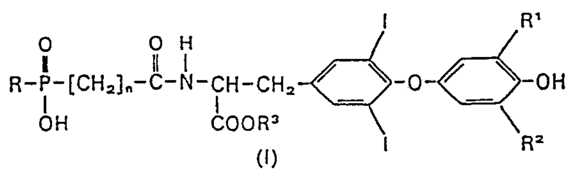

(I)

dans laquelle
n représente un nombre entier compris entre 1 et 6,
R représente un groupe hydroxy, alkyle en $C_1$-$C_6$ ou aryle en $C_6$-$C_{10}$,
$R^1$ et $R^2$ sont identiques ou différents, et représentent un atome d'iode ou d'hydrogène, et
$R^3$ représente un atome d'hydrogène ou un groupe alkyle en $C_1$-$C_6$ ou aralkyle en $C_7$-$C_{10}$,
et sels de celui-ci.

2. Composé de formule I selon la revendication 1, dans lequel
n = 1 à 4,
R représente un groupe hydroxy ou alkyle en $C_1$-$C_4$, et
$R^3$ représente un atome d'hydrogène ou un groupe alkyle en $C_1$-$C_4$.

3. Composé de formule I selon la revendication 1 ou 2, dans lequel $R^3$ représente un atome d'hydrogène ou le groupe méthyle ou éthyle.

4. Composé de formule I selon l'une des revendications 1 à 3, dans lequel au moins l'un des atomes d'iode est radiomarqué en tant que $^{125}I$.

5. Procédé pour la préparation d'un composé de formule I selon la revendication 1, caractérisé en ce que l'on fait réagir un composé de formule II

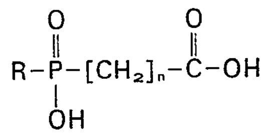

(II)

dans laquelle R et n sont tels que définis dans la revendication 1, ou un dérivé activé de celui-ci, avec un composé de formule III

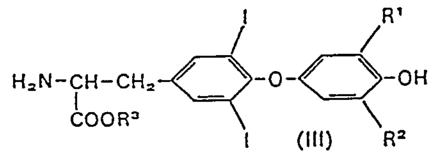

(III)

dans laquelle $R^1$, $R^2$ et $R^3$ sont tels que définis dans la revendication 1, ou éventuellement on utilise un ester de formule III ($R^3 \neq H$), on convertit éventuellement les esters de formule I obtenus ($R^3 \neq H$) en les acides libres de formule I ($R^3 = H$),
et éventuellement on convertit en ses sels le composé de formule I ainsi obtenu.

6. Utilisation d'un composé selon l'une des revendications 1 à 4 dans l'exécution d'un dosage radio-immunologique.

**Revendications** pour les Etats contractants: AT, ES, GR

1. Procédé pour la préparation d'un composé de formule I

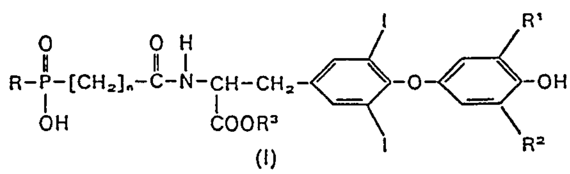

(I)

dans laquelle
n représente un nombre entier compris entre 1 et 6,
R représente un groupe hydroxy, alkyle en $C_1$-$C_6$ ou aryle en $C_6$-$C_{10}$,
$R^1$ et $R^2$ sont identiques ou différents, et représentent un atome d'iode ou d'hydrogène, et
$R^3$ représente un atome d'hydrogène ou un groupe alkyle en $C_1$-$C_6$ ou aralkyle en $C_7$-$C_{10}$,
ainsi que de ses sels, caractérisé en ce que l'on fait réagir un composé de formule II

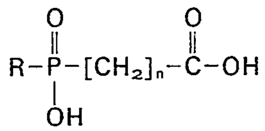

(II)

dans laquelle R et n sont tels que définis plus haut, ou un dérivé activé de celui-ci, avec un composé de formule III

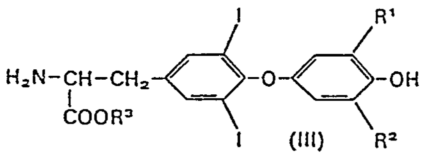

(III)

dans laquelle $R^1$, $R^2$ et $R^3$ sont tels que définis plus haut, ou éventuellement on utilise un ester de formule III ($R^3 \neq H$), on convertit éventuellement les esters de formule I obtenus ($R^3 \neq H$) en les acides libres de formule I ($R^3 = H$), et éventuellement on convertit en ses sels le composé de formule I ainsi obtenu.

2. Procédé selon la revendication 1, caractérisé en ce que l'on prépare un composé de formule I dans lequel

n = 1 à 4,

R représente un groupe hydroxy ou alkyle en $C_1$-$C_4$, et

$R^3$ représente un atome d'hydrogène ou un groupe alkyle en $C_1$-$C_4$.

3. Procédé selon la revendication 1 ou 2, caractérisé en ce que l'on prépare un composé de formule I dans lequel $R^3$ représente un atome d'hydrogène ou le groupe méthyle ou éthyle.

4. Procédé selon l'une des revendications 1 à 3, caractérisé en ce que l'on prépare un composé de formule I dans lequel au moins l'un des atomes d'iode est radiomarqué en tant que $^{125}I$.

5. Utilisation d'un composé préparé selon l'une des revendications 1 à 4, dans l'exécution d'un essai radio-immunologique.